Europäisches Patentamt

European Patent Office

Office européen des brevets

(11) Publication number: **0 328 256**
**A1**

## EUROPEAN PATENT APPLICATION

(21) Application number: 89300508.2

(51) Int. Cl.⁴: **B01J 20/32** , **C12N 11/00**

(22) Date of filing: **19.01.89**

(30) Priority: **21.01.88 US 146678**
**21.01.88 US 146675**

(43) Date of publication of application:
**16.08.89 Bulletin 89/33**

(84) Designated Contracting States:
**BE CH DE FR GB IT LI NL**

(71) Applicant: **OWENS-CORNING FIBERGLAS CORPORATION**
**Fiberglas Tower 26**
**Toledo, Ohio 43659(US)**

(72) Inventor: **Nishioka, Gary Masaru**
**9362 Smoke Road**
**Pataskala Ohio 43062(US)**
Inventor: **Henderson, Mary Beth**
**181 Littleton Road**
**Chelmsford Massachusetts 01824(US)**
Inventor: **Downey, William Edward**
**2650 James Road**
**Granville Ohio 43023(US)**

(74) Representative: **West, Alan Harry et al**
**R.G.C. Jenkins & Co. 26 Caxton Street**
**London SW1H 0RJ(GB)**

(54) **Glass fibers coated with agarose for use as column packing or chromatographic media for bioseparations.**

(57) The invention discloses a glass fiber coated with a porous hydrophilic matrix material comprised of agarose which is derivatized to bind a suitable ligand or biological material. The glass fibers of the present invention are useful in removing contaminates in a chromatographic process. In one embodiment glass fibers have on their surface a coating comprising agarose and polymyxin B which coated glass fibers are useful for removing endotoxins in a bioseparation process.

EP 0 328 256 A1

## GLASS FIBERS COATED WITH AGAROSE FOR USE AS COLUMN PACKING OR CHROMATOGRAPHIC MEDIA FOR BIOSEPARATIONS

This invention relates to the use of glass fibers coated with agarose for use as column packing or chromatographic media for bioseparations.

Biotechnology products are now being commercially manufactured by various types of biological or synthetic processes. These biotechnology products must be purified to remove contamination from the final product. Examples of biotechnology products which need to be purified include enzymes, blood products, regulatory proteins, drugs such as vaccines, and monoclonal antibodies.

Purification costs for new biotechnology products normally account for approximately 30-70% of the manufacturing costs. As such, the rapidly growing field of biotechnology is demanding increasingly more efficient and economic means for biological product recovery and isolation. For example, the expected commercialization of recombinant DNA products is overwhelming and their potential uses have generated a high interest in large scale chromatographic separations. However, chromatographic separation and purification of biological materials is a difficult and costly process within the biotechnology industry. The advancement of biotechnology and commercialization of DNA products will, in part, be contingent upon the development of improved materials useful for bioseparation processes which provide greater pH stability, allow increased throughput, and maintain rapid absorption/desorption kinetics.

Of particular concern in the separation and purification of biological materials is the removal of endotoxins which are often generated by the new biotechnology processes. It is especially important for biotechnology products produced by gram negative bacteria to be purified since endotoxins, which are fragments of gram negative bacterial cell membranes, are commonly found in aqueous solutions containing the final product. In particular, endotoxins are a special concern among the companies that manufacture and sell injectable products for human or animal use. If the endotoxins are injected, even at extemely low concentrations, the endotoxins cause elevated body temperatures. Therefore, it is important that the endotoxins are removed from drug preparations. Of particular concern are injectable products which are produced by bacterial fermentation. Purification of these products must include endotoxin removal to concentrations below approximately 0.1 ng/ml.

The endotoxin-removal systems must meet several criteria including capturing sufficient levels of endotoxin during the manufacturing process while not capturing any other materials other than the endotoxins. It has been found that various currently available endotoxin-removal systems tend to generally capture negatively charged materials. This phenomenon is called non-specific adsorption and is undesirable in the manufacturing of biotechnology products.

Most manufacturing processes currently rely upon the inadvertent removal of endotoxins during the separation and processing steps. Such inadvertent removal systems include processes which incorporate anion chromatography, ultrafiltration, precipitation or thermodistillation. For example, endotoxins are currently removed from pharmaceutical preparations by membrane filtration. However, there are problems with this endotoxin-removal system since membrane filtration is slow, expensive, and will not work if the pharmaceutical product is a protein with a molecular weight within a factor of 10 of the endotoxin. Therefore, a combination of methods, in addition to membrane filtration, is required in order to obtain sufficient product purity. As such, the removal of endotoxin by these methods is not always reliable or complete. Often, manufacturers have been required to either discard or reprocess entire batches of product which do not pass final quality control tests because the level of endotoxins in the product was too high.

In addition, some manufacturers inadvertently remove endotoxins in other process steps, primarily during ion exchange. However, additional problems develop when the desired biotechnology product has the same physical characteristics as the endotoxin. Still further problems are also likely to occur as the number of processing steps is reduced. While affinity chromatography can bring about a single step purification procedure, the commercial acceptance of affinity chromatography has not materialized mainly due to the high cost and the manufacturer's reliance on traditional purification techniques. Therefore, there is a need for an endotoxin removal system designed to specifically remove traces of endotoxin without adversely affecting the final product. Such a system would ensure manufacturers of producing a better quality product and would reduce the time lost when batches of product need to be discarded or reprocessed.

There are several criteria that must be met for acceptance of an endotoxin removal system. First, the support/ligand matrix used to remove endotoxins must not leach. Nor must it absorb other proteins. Further, the system must display sufficient reduction of endotoxin in the final product, preferably a log reduction to achieve less than .1 ng/ml in the final product. These criteria are complicated by the range of various process conditions which are encountered among the various manufacturers, such as, for example, volume of process stream ranging from 1-1000 liters, flow rates of 10 ml. to 6 liters per minute. pH ranges of 2.0 to 12.0, and endotoxin concentrations of up to 100 ng/ml.

Certain antibiotics, such as polymyxin which is a small peptide, are known to have an affinity for endotoxins. Their use as a selective endotoxin adsorbent requires a support material to which the anitbiotic can be attached for contact with the endotoxins during the removal process. Therefore, there is a specific need for a support material for use as an adsorbent for endotoxin removal wherein the phenomenon of antibiotic affinity for endotoxins can be utilized advantageously.

There is also a need for a system to purify the biotechnology products manufactured using the various known biological and synthetic processes, which system can be specifically adapted or derivatized to meet the particular manufacturer's needs. This invention is also directed to this need.

This invention is directed toward an improved separation material which will enable a large scale commercial chromatographic separations to be economically performed.

This invention relates to an improved material for use in purifying biotechnology products manufactured using either biological or synthetic processes. In particular, the invention relates to a glass fiber material having a relatively high external surface area coated with agarose, a porous hydrophilic matrix. The coated fiber can be derivatized such that any one of the many known ligands or biological materials can be bound to the coated glass fibers.

The agarose-coated glass fibers are used as generic supports for biochemical separations. The agarose is porous and can be derivatized to bind various ligands or biological materials for use in affinity chromatography. In particular, agarose is dispersed in water and heated in order to form a hot viscous solution. The solution is coated onto glass fibers either during the glass fiber-forming process or after the glass fiber-forming process. The agarose cools onto the glass fiber surface adhering to the glass fiber's surface.

In one embodiment the invention relates to the immobilization of an antibiotic, such as polymyxin B, on agarose-coated glass fibers for use in removing endotoxins in a chromatographic process. A material contaminated with endotoxins is passed over the agarose-coated glass fibers derivatized with the antibiotic. The endotoxins are removed from the material by adsorption onto the agarose-coated glass fibers.

The agarose-coated glass fibers are easy to disperse and give excellent flow properties. The low packing density obtainable with glass fibers makes chromatography processing at high throughputs possible. The excellent mechanical properties of the glass fibers also contribute favorably to the flow properties.

The agarose-coated glass fibers of the present invention are also useful in high performance filter units for use in hospitals for such uses as high-performance disposable filter units for use in dispensing I.V. solutions and disposable filter units for use by hospital pharmacies in extemporaneous compounding. Whether I.V. solutions are purchased pre-mixed from a manufacturer, or whether a hospital's pharmacy creates its own I.V. solutions, ("extemporaneous compounding"), the potential for endotoxin contamination by microorganisms is real. Hospitals often use in-line filters connected to I.V. solutions in an attempt to prevent bacteria and particulates from entering the patient's bloodstream, but these in-line filters do not always prevent endotoxins present from being released.

Further, there is a demand for low-endotoxin cell culture media for use by biotechnology and pharmaceutical companies to produce a variety of recombinant DNA pharmaceuticals and vaccines. Cell culture media manufacturers are interested in an improved endotoxin removal system. The coated glass fibers of the present invention are also useful in the cell culture media market.

Fig. 1 is a schematic diagram of one of the endotoxin removal systems.

Fig. 2 is a schematic diagram of another of the endotoxin removal systems.

Fig. 3 is a graph depicting the breakthrough profile for endotoxin adsorption in a column test.

Fig. 4 is an enlargement of the graph shown in Fig. 3.

Fig. 5 is a graph depicting the breakthrough profile for endotoxin adsorption by agarose-coated polymyxin activated glass fibers.

According to the present invention, well-dispersed, coated glass fibers are used as effective chromatographic supports in the purification of biological products. The coated glass fibers have an advantage over commercial gel supports in high throughput, high flow rate applications due to the glass fibers' superior mechanical strength and high external surface area. Further, the activated coated fibers display low levels of non-specific absorption and remove significant quantities of contaminants from the solution containing the final product. In particular, the high external surface area and noncompressible nature of glass fibers yield significant flow advantages giving higher throughput in a chromatographic column separation. Also, control .

of the glass fiber diameter and coating thickness yield advantages in resolution and column flow.

This invention can be practiced with any glass fibers which can be formed and chopped into suitable lengths. The term "glass fibers" as used herein shall mean filaments formed by attenuation of one or more streams of molten glass and to short lengths of glass formed when such glass fiber filaments are cut or chopped. In a preferred embodiment of the invention, randomly oriented, unsized, chopped glass fibers having a length of approximately 1/4" are used. Glass fibers have such advantages as noncompressibility, high external surface area, macroscopic lens, microscopic diameter and hydrophilic surfaces. The packed glass fibers give good flow dispersion characteristics. The glass fibers perform well at high flow rates where conventional chromatographic media normally fail. Agarose-coated glass fibers show no measurable amount of non-specific adsorption with model proteins.

The randomly packed glass fibers have a low density (5-10%) compared to spherical packing materials (typically 50%). The low packing density obtainable with the glass fibers makes processing and purification of the biotechnology product at high throughputs possible. Uniform dispersion of fibers during the column packing aids in achieving column flow without significant channeling occurring through the packed fibers.

The present invention can also be utilized as a cartridge design chromatography column which is simple to use in that the entire cartridge can be inserted into the production process by the manufacturer.

In addition, the agarose-coated glass fibers can be formed into a glass cloth for use as an adsorptive filter to be used to remove particulates by filtration, as well as dissolved material by adsorption.

By the use of the present invention it is possible to immobilize a wide variety of biological materials to produce novel composites. These biological materials can include animal cells, anitbodies, antibiotics, antigens, carbohydrates, bacteria, coenzymes, enzymes, fungi, microbial cells, plant cells, tissue cultures or yeast. The following examples describe the process for making and the manner of use of the invention with respect to the immobilization of polymyxin B on agarose-coated glass fibers for use in removing endotoxins. For example, endotoxins are absorbed from a flowing process fluid onto activated coated fibers. The feed endotoxin concentration is reduced from 1700 ng/ml to below .1 ng/ml. The capacity of the activated coated fibers is 133,000 ng endotoxins/g glass.

The chromatographic medium of the present invention is useful for removal of endotoxins from products produced by conventional and r-DNA technology and purification of blood proteins such as antithrombin III, Factor 8, and albumin. For each specific application the agarose-coated glass fibers are derivatized with a material having a specific affinity for the product to be separated.

Several requirements which must be met for the chromatography process to be commercially acceptable include that the requirement of the system must not leach, must not adsorb other proteins and must reduce endotoxins to less than .1 nanogram/ml in the final product. According to the present invention leaching of biological materials was not detected down to the part per thousand level.

The glass fibers are coated with agarose as follows: the agarose is dispersed in water, preferably 2-4 percent, by weight, and heated to approximately 70°C in order to form a hot viscous solution. The agarose solution is coated onto the glass fibers either during the glass fiber forming process or after the glass fiber forming process. The agarose-coated glass fibers are allowed to cool and the agarose cools onto the glass surface, adhering to the glass surface through multiple hydrogen bonds. The agarose-coated glass fibers thus form a biologically inert material having a relatively uniform size.

The agarose-coated glass fibers are useful in a chromatographic separation process such as affinity chromatography or liquid chromatography. The agarose-coated glass fibers can be used to immobilize biomaterials for an end user's own separation needs. The agarose-coated glass fibers can be derivatized with an immobilized antibody, antibiotic ligand or protein designated to separate or purify specific products. The agarose-coated glass fibers can be provided in a cartridge design that is to be added to a manufacturing process stream with virtually no modifications to the manufacturing process.

The immobilization of polymyxin B on glass fibers can be accomplished as follows: Water sized EK 6.75 glass strand is coated off-line with a 4% agarose 1$H_2O$ solution. The coated strand is rinsed with a deoxycholate (1M) solution, followed by a pyrogen-free $H_2O$ rinse, to assure that the strands are uncontaminated prior to cyanogen bromide (CNBr) activation.

The agarose-coated glass fiber strands are activated by treatment with a 100 mg/ml solution of CNBr in 2M phosphate buffer, pH≈11, for 1 hour; after which the glass is rinsed with pyrogen-free $H_2O$ and then with 0.5M NaCl in 0.1M NaHCOH3, pH≈9.0. Polymyxin≈(200 mg/ml in 0.5 NaCl/0.1M NaHCO₃) is allowed to react overnight at 4°C. The excess polymyxin is rinsed out using pyrogen-free $H_2O$ and any unreacted imido carbonate sites are blocked by a 1 hour treatment with a 1M glycine solution. At this point the coated glass fibers are rinsed with pyrogen-free $H_2O$ and can be stored at 4°C in $H_2O$ or deoxycholate.

The following are examples of how the glass fibers can be coated:

Aqueous solutions of 2 percent and 4 percent agarose (Sigma #A-3768) are coated onto J-7 strand glass fibers using a hot melt chemsize applicator. The solution temperature is kept at approximately 80° ± 5° C, and the applicator temperature is approximately 60° ± 5° C. In a preferred embodiment the agarose is heated to approximately 70° C.

Packages composed of 400 filaments/strand were also prepared by collecting a split strand. The coating weight on these samples are listed in Table I, as measured by loss on ignition tests (LOI). Coating weights of up to at least about 1 percent agarose of the total weight are achieved. As seen in Table I, the percent, by weight, of agarose coating can range between 2-4%.

Higher weights of agarose are achieved using an offline coating process. The glass strand is dipped in a agarose solution, sent through a stripper dye, and wound on a spool. Table II contains information on the coating weight of these samples.

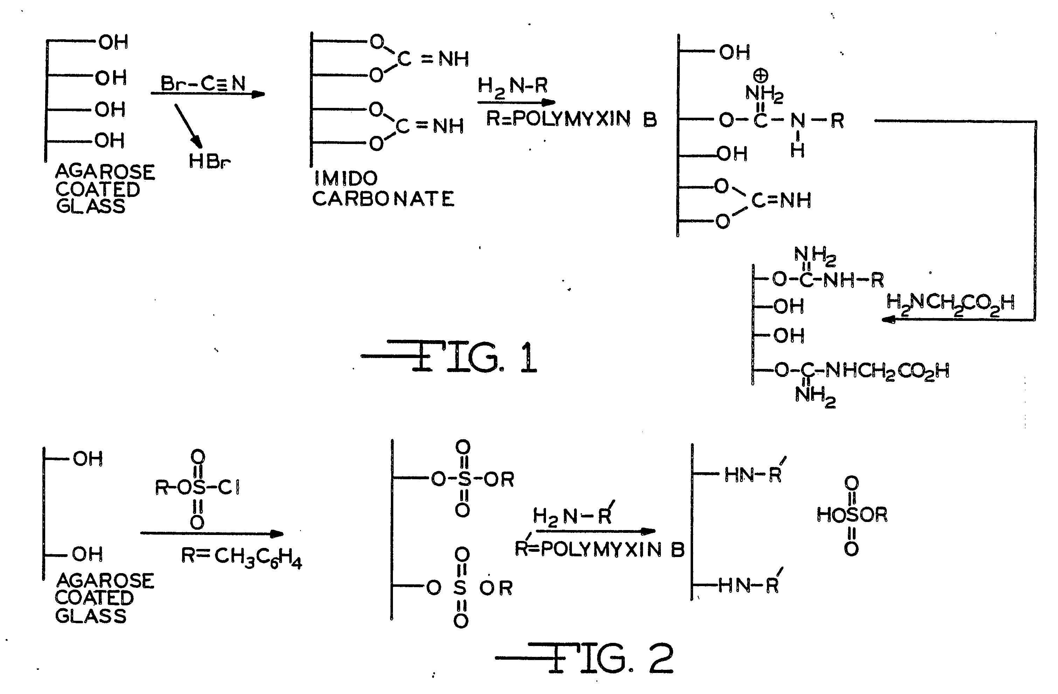

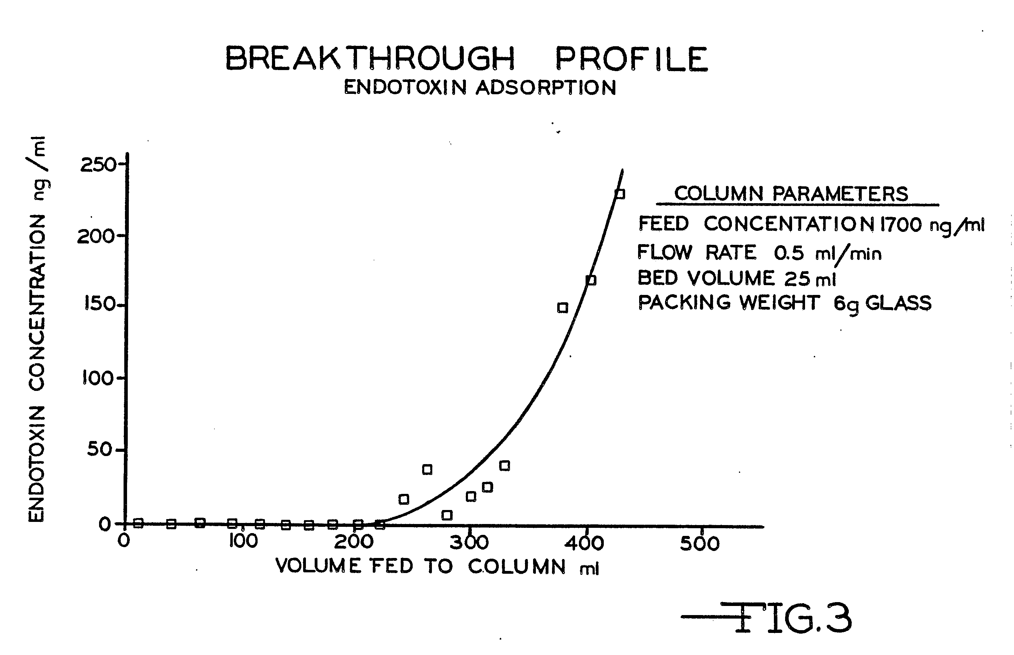

Figures 1 and 2 are schematic diagrams of the endotoxin removal system. Glass fibers are coated with agarose, a porous, hydrophilic gel. The function of the agarose is to provide increased surface area and a biologically inert surface.

Two attachment chemistries are shown: activation with cyanogen bromide, as shown in Fig. 1 or activation with a sulfonyl chloride, as shown in Fig. 2. In cyanogen bromide activation, vicinal diols in the agarose react to form a relatively stable imido carbonate active intermediate. When attacked by an amine (the polymyxin B) under nucleophilic conditions, the amine (polymyxin) becomes immobilized via an isourea linkage. The bond forms rapidly and is stable at neutral pH.

The derivatization procedure for CNBr was as follows: Stock solutions were prepared 100 mg/ml CNBr in 2M potassium phosphate buffer (pH 11.5-12.0); 1M $NaHCO_3$ + .5M NaCl (pH 8.3); 1M Glycine in tris buffer (pH 8.0); 2M Potassium phosphate buffer (pH 11.5-12.0); 200 mg/ml Polymyxin B sulfate in .1M $NaHCO_3$ + .5M NaCl (pH 8.3); .1M Tris buffer (pH = 8.0) + 1% sodium deoxycholate (deoxycholic acid).

At a rate of 0.05ml CNBr stock solution of 5 ml $PO_4$ buffer enough solution was prepared to completely cover the sample (in a sterile container). The sample was allowed to soak, while stirring, for 1 hour in the CNBr/phosphate solution, and then was rinsed 3 times with pyrogen-free water and one time with .1M $NaHCO_3$ + 1.5M NaCl. The sample was immersed in the 200 mg/ml Polymyxin solution and allowed to soak 24 hours. The solution was stirred or agitated at least part of the this time and was kept on ice or in the refrigerator. The sample was then rinsed 3 times with pyrogen-free water and then immersed in 1M glycine solution for 1 hour; further rinsed 3 times with pyrogen-free water and soaked in deoxycholate solution until ready to test; then rinsed 3 times with pyrogen-free water immediately prior to testing for endotoxin removal.

Sulfonyl chlorides are also used to activate the agarose, as shown in Fig. 2. The sulfonyl chloride reacts with a hydroxyl group on the amine to form the sulfonate ester. The sulfonate ester can be tailored to be more or less reactive by choosing a sulfonyl chloride having a different electron withdrawing groups. The resulting intermediate formed can then be more or less reactive, as is desired. The sulfonate esters are subject to attack by primary amines under acidic or basic conditions and the resulting linkage is a secondary amine group. Activation with sulfonyl chloride results in a bond (secondary amine) which is impervious to hydrolytic attack and which is unchanged over a broad pH range. One advantage to the use of the sulfonyl chloride chemistry is that the sulfonyl chloride compounds are less hazardous to handle than other compounds, such as, for example, cyanogen bromide.

Four different sulfonyl chlorides; p-nitrophenyl, tosyl, pentafluorobenzyl, and triisopropyl benzyl, were tested for their ability to activate the agarose. Of the four, p-nitrophenyl gave the highest subsequent levels of loading, followed by triisopropyl benzyl, tosyl and pentafluorobenzyl. The pentafluoro sulfonate ester intermediate formed was very reactive.

Activation with a sulfonyl chloride requires the use of an aprotic solvent. In the first tests the activations were done in 5% triethylamine/ethylacetate. The triethylamine acts to sequester the HCl produced during the reacting by forming the insoluble triethyl ammonium chloride salt. In further tests, a number of other solvents were tried-pyridine, dioxane, methylethyl ketone, THF. Although the agarose does not swell in pyridine as much as it would in water or ethanol, pyridine produces a greater degree of swelling than any of the other solvents.

In a typical activation procedure, 25 grams of agarose which had been dried under vacuum in a dessicator and 12.5 grams of pNPh are dispersed in 100ml of pyridine. (No triethylamine is needed, as the pyridine acts as the acid scavenger). The reaction mixture was shaken gently. The pyridine was filtered and the agarose was suspended and completely dispersed in distilled water. The agarose was filtered by gravity and the water wash was repeated. The agarose was finally rinsed with one liter of distilled water using suction.

The activated agarose was checked for active sites by allowing 0.1g (wet) of material to soak for 2 hours in 5% 1,6-diamino hexane/0.1M NaHC $NaHCO_3$ (pH 11.0) solution, thoroughly rinsed, with distilled water and checked with ninhydrin. A deep purple color indicates extensive activation of the agarose by the sulfonyl chloride. The agarose can be stored damp at 4°C for at least 3 months without loss of activated sites.

Referring now to Table I, Sample No. 1 (bare glass) and Sample No. 5 were tested for endotoxin removal efficiency in batch tests. Sample No. 5 was reacted with CNBr, polymyxin, and glycine. The batch tests involved immersing 20 mg. of glass in 10 ml of endotoxin solution. The endotoxin solutions tested had a concentration of 1, 10, 100, 1000 and 10,000 ng/ml. Sample No. 1 did not remove endotoxin from all solutions. Sample No. 5 removed 99 percent of available endotoxin from all solutions except the 10,000 ng/ml after 24 hours. Sample No. 5 removed less than 90 percent of the endotoxin from the 10,000 ng/ml solution. These results indicate that even thin coatings removed appreciable endotoxin. The capacity, C, of Sample No. 5 is estimated to be: 9900 ng$<$C$<$90,000 ng or, normalized per gram of glass: 4.95 x $10^5$ ng$<$C/g$<$4.5 x $10^6$ ng.

Table II shows that different types of glass fibers can be effectively coated with agarose. Sample No. 3 in Table II was wound on a stainless steel tube which had holes drilled through it. Endotoxin solution was pumped into the tube, forced out the holes and through the densely packed strand. At a flow rate of 1.5 column volumes/hr., 99 percent of the endotoxin was removed.

In the purification of biological materials, great care must be taken to avoid the problems of leaching, which is the introduction of foreign material into the process stream. Leaching occurs when compounds or ions of the chromatographic material are soluabilized by the process stream, creating a potential source of contamination. In an endotoxin removal system, there are three leachable components: the antibiotic or an amino acid fragment of the antibiotic, agarose, and inorganic ions from the glass fibers. The inorganic ions are not a problem when the agarose completely coats the glass surface. Further, any ions leached from the glass surface are detectable by routine ion analysis.

Referring now to Table III, four different methods were used to detect the antibiotic polymyxin at low levels of concentration--UV spectroscopy, the ninhydrin test, Commassie Blue staining, and the BCA assay for proteins sold by the Pierce Co. At a lower limit of 1 g/ml, polymyxin at the parts per thousand or $10^{-6}$ M level is detectable in solution. In order to increase the sensitivity level for polymyxin and to establish a method for the detection of low levels of agarose in solution, samples were tested for LC-FID (Liquid chromatography-flame ionization detection). Through the use of direct probe mass spectroscopy both polymyxin and agarose were detected at the 100 ng/ml level.

Referring now to Table IV, further experiments were conducted to test the integrity of the coatings during and after a total of 25 column volumes of water or buffer. All samples consisted of 1 gram of glass and a column volume was defined as 1 ml of solution. Each of 5 different samples was washed with five column volumes of endotoxin free water (efw). For the polymyxin and agarose tests the buffers were 0.1 M phosphate at pH 4.5, 7.0 or 9.0. For the inorganic ion tests, endotoxin free water (efw) was pHed to 4.5 with acetic acid (HOAc) and 9.5 with ammonium hydroxide (NH$_4$OH). Endotoxin free water was used for the pH 7.0 assays.

The first column volume of buffer was collected, along with the 15th column volume of buffer. A 5-column volume of efw rinse was then passed through the column and the final 1 ml of solution was collected. These samples were then subjected to the BCA test for proteins. If interference caused by the buffering ions occurs, lyophilizing the samples (HOAc and NH$_4$OH are both volatile buffers) and resuspending them in efw alleviates the interference.

The BCA test was performed on the samples collected and the results appear in Table IV. No detectable levels of polymyxin were evident in solution under any of the buffering conditions. Controls run on the buffers showed that the buffers did not interfere with the assay conditions. These results indicate that down to the ppt level no polymyxin is detectable in solution.

The procedures described above are used to prederivatize agarose with sulfonyl chloride. Optimum coating properties may include, for example, thin coatings which more efficiently remove endotoxins from solution at high throughput; thick coatings which best remove endotoxin at lower throughput.

The following examples are given to show various uses of the coated fibers of the present invention. These examples given are not meant to be construed in a limiting manner and various other uses are contemplated to be within the scope of this invention.

EXAMPLE 1

Polymyxin activated coated fibers of the present invention are useful as an endotoxin specific adsorbent. The coating material tested for endotoxin adsorption was agarose. This material was examined both with and without polymyxin activation. Results of these experiments, obtained using flasks containing a known amount of adsorbent material and an endotoxin laden fluid, are shown in Table V. One result shown by these tests is that the polymyxin activated materials adsorbed significantly more endotoxin than the non-activated materials.

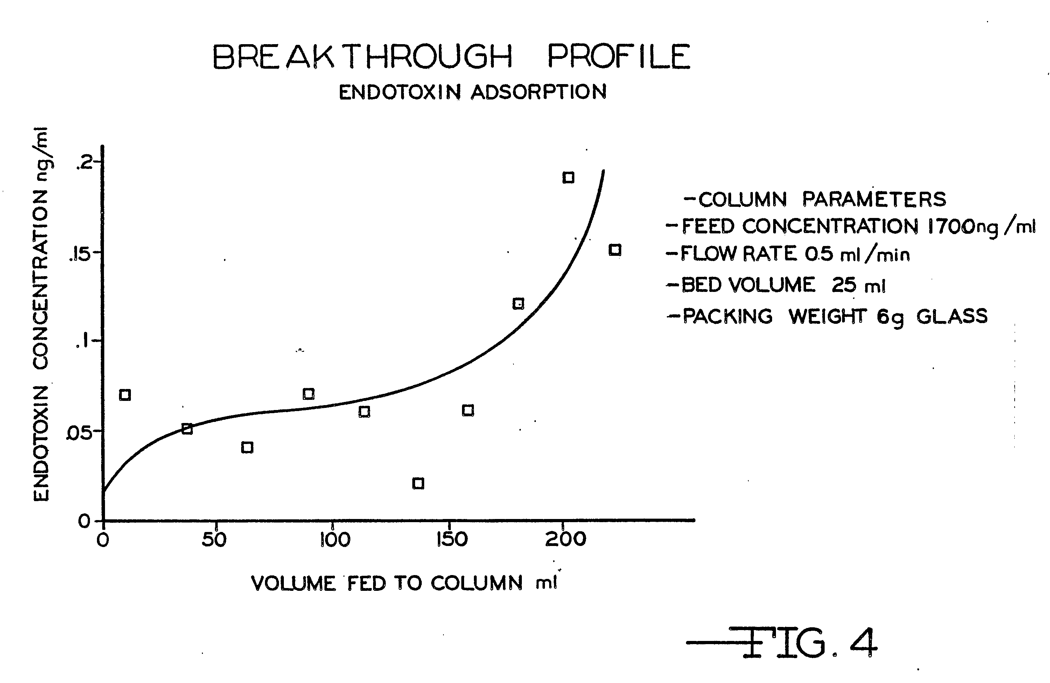

A column test was conducted in which endotoxin was adsorbed from a flowing process fluid onto polymyxin activated agarose-coated fibers. Shown in Figure 3 is the breakthrough profile of endotoxin adsorption comparing the endotoxin concentration to the volume fed to the column. In this test the feed concentration was 1700 ng/ml, the flow rate was 0.5 ml/min., the bed volume was 25 ml, and the packing weight was 6 g. glass. Figure 4 is an enlargement of the initial portion of the graph shown in Fig. 3. This material was effective in reducing the feed endotoxin concentration from 1700 ng/ml to below 0.1 ng/ml. This is an excellent performance in terms of percentage endotoxin removed and, the reduction of endotoxin concentration from an unacceptably high level in the feed to less than 0.1 ng/ml in one pass. The estimated total capacity for this material, from an extrapolation of the breakthrough curve, is 133,000 ng endotoxin/g glass fiber. Assuming a maximum allowable endotoxin concentration of 0.1 ng/ml, the effective capacity for this column was 49,000 ng endotoxin/g glass.

## EXAMPLE 2

The adsorption of endotoxin on bare and coated glass fiber materials was examined in a series of small scale batch experiments. A column test was performed with one of these materials (polymyxin activated agarose) to obtain a breakthrough profile. Endotoxin concentration in solutions was determined using the Chromogenic quantitative LAL Assay Procedure (Whittaker, Inc.).

Small scale batch endotoxin adsorption tests were conducted: Stock endotoxin solution was prepared using Lipopolysaccharide No. L-2637 From E. coli 055:B5 (Sigma Chemical Co., St. Louis, MO). The desired amount of fiber material was placed in endotoxin free glass tubes (200 mg for agarose-coated fibers). Stock endotoxin solution was diluted to desired initial concentrations and then, 5 ml was added to each tube containing the fiber materials. Fiber materials were contacted with the endotoxin solutions for 30 minutes at room temperature following vigorous mixing by vortexing. At the end of the contact time, the supernatant was diluted to the assayable range for endotoxin (0.01-0.1 ng/ml) and assayed.

Small scale batch endotoxin adsorption test results were as follows: For the agarose-coated fibers, the specific adsorption of endotoxin due to polymyxin was compared with the non-specific adsorption. Results are given in Table VI. As can be seen, the agarose-coated glass fibers activated with polymyxin adsorbed greater amounts of endotoxin per fiber weight (ng/mg) than the agarose-coated fibers without polymyxin.

## EXAMPLE 3

The procedures used to prepare a column and conduct an adsorption test are as follows: Agarose-coated glass fibers were packed into a 2.5cm diameter glass column in the same manner as for the flow characterization studies. Bed height was 10 cm. Column and associated tubing from pump intake to fraction collector were washed by passing approximately 10 bed volumes of 1% doxycholate solution followed by 20 bed volumes of endotoxin-free water. A flow rate of approximately 3 ml/min was used for washing. The column effluent was tested to insure that it was free of endotoxin and deoxycholate. At this point, the column was considered free of endotoxin and ready for the adsorption test. An endotoxin containing feed solution was passed through the column at a selected flow rate. Effluent fractions (20 ml) were collected into endotoxin-free glass tubes using an automated fraction collection. The feed and collected fractions were assayed for endotoxin.

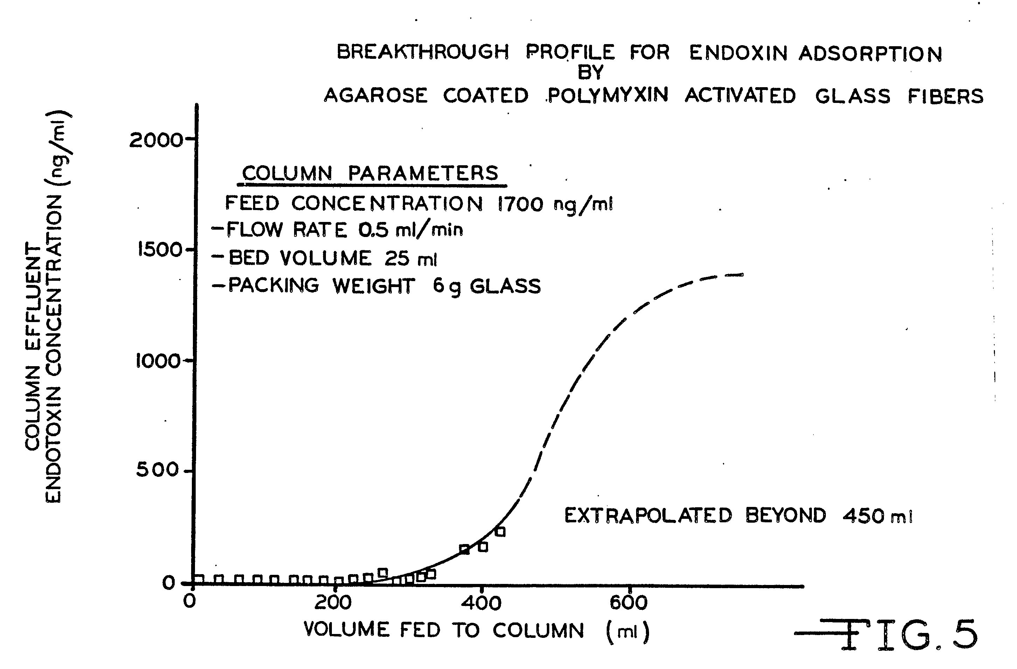

The breakthrough profile for removal of endotoxin by agarose-coated, polymyxin activated glass fibers is shown in Fig. 5. In this test, the feed concentration was 1700 ng/ml, the flow raate was 0.5 ml/min., the bed volume was 25 ml, and the packing weight was 6 g. glass. From this, the total and effective endotoxin capacities were estimated for agarose-coated fibers. Total capacity is the amount that can be adsorbed if all available sites are utilized. The effective capacity is defined as the amount of endotoxin adsorbed prior to the effluent concentration exceeding an allowable maximum, as shown in Table VII.

## EXAMPLE 4

The non-specific adsorption of protein by the agarose-coated fibers was examined using insulin and bovine serum albumin (BSA). The Pierce Chemical Co. Protein Assay was used to measure protein concentration. None of the fiber samples tested contained polymyxin in the coating. These experiments were performed in a similar manner to the small-scale batch endotoxin adsorption tests. The desired quantity of bare or coated fibers was weighed and placed in glass tubes. Protein solution (5-10 ml) was added and allowed to contact 30 minutes with occasional agitation. The amount of protein adsorbed was determined by assaying the solution after contacting and calculating the difference from the starting solution.

Protein adsorption of bovine serum albumin (BSA) and insulin occurs on both coated and bare glass fibers. When the bulk phase concentration is low, no adsorption was detected. This is due to the insignificant amount of protein removed from the bulk phase. Table VIII gives the protein adsorption results for the materials tested.

## EXAMPLE 5

A commercial sample of polymyxin B immobilized on agarose was loaded into a column and tested on a chromatograph test endotoxin removal as a function of space velocity. Column tests were run on commercial samples of polymyxin B immobilized on agarose. Removal of endotoxin as a function of flow rate was measured and was provided as a baseline against which test fibers were compared. Column testing of glass fibers show that the samples removed 100 percent of the endotoxin at space velocities of 0.1 and 0.5 per hour.

## EXAMPLE 6

Glass fibers were coated with agarose using an off-line coating process. The agarose-coated glass strands were soaked in a 1% deoxycholate solution at pH = 8 after curing in order to remove endotoxins picked up during the coating trial. Batch tests for samples coated in the off-line coating trial showed that samples soaked in the deoxycholate solution did not lose activity.

## EXAMPLE 7

Various changes can be made in the coating procedure such as after addition of the cross-linking solution, the strands are placed in a clean hood and allowed to cure at room temperature for 4 hours. The strands are then soaked in a 1% deoxycholate solution, (pH = 7) to remove endotoxins trapped during the coating process. High activities can be obtained using this modified coating procedure. The strands were off-line coated and the higher activities were obtained in batch tests. The strands were loaded into columns. It was found that no contamination was introduced when endotoxin free water was passed through the column.

## EXAMPLE 8

Extended test results show that in an 8-hour period a column containing agarose-coated glass fiber strands immobilized with polymyxin B removed over 99 percent of endotoxin from a solution at space velocities from 0.25 to 10 hour $^{-1}$. At various flow rates and times, over 99% of the endotoxin was removed. These results reveal that the actual capacity of a 40 ml column for endotoxin is over 1.5 mg, since the column was not regenerated in the 40-hour test. Typical industrial solutions will contain 1-1000 nanograms per ml.

## EXAMPLE 9

A second testing of coatings included use of various strands, which were coated off-line with agarose. These coatings were heavy and comprised from 30 to 50% of the total weight of the system. The coated strands were then activated using cyanogen bromide. The activated strands comprise a "generic" support that can bind any protein. The activated strands were then soaked in polymyxin which binds to the cyano groups on the agarose surface.

## EXAMPLE 10

A portion of the polymyxin-agarose-glass composite was used for protein tests which qualitatively measure the presence of immobilized polymyxin. The remaining strands were washed in glycine (an amino acid) to neutralize any unreacted cyano groups. These strands were then measured for endotoxin removal activity. The fibers produced selectively removed more than 99% of the endotoxin from the solution with little non-specific adsorption. A solution containing 1700 ng/ml was purified to endotoxin level of less than .1 ng/ml. The capacity of the column was about $10^6$ nanograms. The concentration and the time of exposure of the agarose-coated glass to the coupling agent was controlled. Good results are obtained when each gram of glass is immersed in 10 ml of a solution of 1 to 4 mg coupling agent/ml for 1 to 4 hours.

## EXAMPLE 11

In another test, glass fiber strand was off-line coated with agarose then chopped and dispersed to form a slurrry. The slurry was poured into a column, then derivatized and polymyxin was attached to the column packing. The glass strands which had been coated with agarose and loaded with polymyxin using cyanogen bromide activation were then tested for endotoxin removal. The glass strand samples removed between 50 to 90% of the applied endotoxin.

## EXAMPLE 12

p-Nitrobenzene sulfonyl chloride was used to activate glass samples which had been treated with bis(2-hydroxyethyl) aminopropyltriethoxy-silane. These samples were allowed to react with polymyxin and tested for ability to remove endotoxin from solution. The glass strand treated as above removed between 95% to 99.5% of the applied endotoxin.

Further, tests show that more uniform agarose coatings may be possible by altering the coatings during the forming process. In single-hole bushing experiments, agarose was coated onto a glass strand and was found to be satisfactory after derivatization for removing endotoxin.

It will be evident from the foregoing that various modifications can be made to this invention. Such, however, are considered within the scope of the invention.

TABLE I

| Sample | Description | % Agarose | Pump Speed | Roll Speed | Fila./Strand | % LOI |
|---|---|---|---|---|---|---|
| 1. | H$_2$O sized control | 0 | | 6 | 2000 | .13 |
| 2. | Agarose coating | 2 | | 6 | 400 | .19 |
| 3. | Agarose coating | 2 | | 6 | 2000 | .26 |
| 4. | Agarose coating | 2 | | 12 | 400 | .76 |
| 5. | Agarose coating | 4 | | 6 | 400 | .87 |
| 6. | Agarose coating | 4 | | 6 | 2000 | .32 |
| 7. | Agarose + sulfonyl chloride | 3 | | 6 | 400 | .31 |
| 8. | Agarose + sulfonyl chloride | 3 | | 6 | 2000 | .25 |
| 9. | Agarose | 4 | 30 | 6 | 400 | 10.03 |
| 10. | Agarose | 4 | 20 | 3 | 400 | .95 |
| 11. | Agarose | 4 | 20 | 6 | 400 | 1.80 |
| 12. | Agarose (no prepad) | 4 | 30 | 6 | 400 | 1.00 |
| 13. | Agarose (no prepad) | 4 | 30 | 6 | 400 | .91 |

TABLE II

| Coating weights, offline | | |
|---|---|---|
| Sample | % Agarose | % Wt. Loss |
| 1. E glass cloth | 2 | 50.7 |
| 2. S glass cloth | 2 | 41.1 |
| 3. EK6.75 strand | 2 | 25.4 |
| 4. SG75 strand | 2 | 44.9 |

TABLE III

| Detectability of polymyxin in solution | | | | |
|---|---|---|---|---|
| Concentration Polymyxin | UV | Ninhydrin | Commassie Blue | BCA[a] |
| 10 mg/ml | + | + | + | + |
| 1 mg/ml | + | + | + | + |
| 100μg/ml | - | +b | + | + |
| 10 μ g/ml | - | - | - | + |
| 1 μ g/ml | - | - | - | + |

(a) 1 ml samples concentrated by lyophilization.
(b) 5 coats of sample applied to plate.

## TABLE IV

| BCA Assay of sample in Leaching Experiments | | 1st Col. Volume | 15th Col. Volume | Final Rinse |
|---|---|---|---|---|
| H₂O sized fibers | pH = 4.5 | - | - | - |
| | pH = 7.0 | - | - | - |
| | pH = 9.0 | - | - | - |
| Agarose-coated fibers | pH = 4.5 | - | - | - |
| | pH = 7.0 | - | - | - |
| | pH = 9.0 | - | - | - |
| Agarose/CNBr*/polymyxin coated fibers | pH = 4.5 | - | - | - |
| | pH = 7.0 | - | - | - |
| | pH = 9.0 | - | - | - |
| Agarose/SCl/polymyxin coated fibers | pH = 4.5 | - | - | - |
| | pH = 7.0 | - | - | - |
| | pH = 9.0 | - | - | - |

*CNBr = cyanogen bromide
SCl - sulfonyl chloride

## TABLE V

| Endotoxin Adsorption On Coated Glass Fibers | | | | |
|---|---|---|---|---|
| Sample | Polymyxin Activated | Initial Endotoxin Conc. NG/ML | Final Endotoxin Conc. NG/ML | Endotoxin Adsorbed NG/MG Fiber |
| Agarose Coated | No | 400 | 340 | 1.5 +/-0.3 |
| Agarose Coated | No | 400 | 320 | 2.0 +/-0.3 |
| Agarose Coated | Yes | 780 | 310 | 11.8 +/-1.1 |
| Agarose Coated | Yes | 780 | 430 | 8.8 +/-1.1 |

11

### TABLE VI

| Endotoxin Adsorption On Glass Fiber Support Materials | | | | | |
|---|---|---|---|---|---|
| Coating | Fiber Weight (mg) | Vol. (ml) | Endotoxin Initial (ng/ml) | Conc. Final (ng/ml) | Endotoxin Adsorbed (ng/ml Fiber) |
| Bare Glass Fibers | 10 10 10 10 | 5 5 5 5 | 3760 37.6 3.76 .376 | 3240 29.5 3.5 .295 | 260 4 .13 .04 |
| Agarose-coated fibers without polymixin | 200 200 | 5 5 | 400 400 | 340 320 | 1.5 +/-0.3 2.0 +/-0.3 |
| Agarose-coated fibers with polymixin | 200 200 | 5 5 | 780 780 | 310 430 | 11.8 +/-1.1 8.8 +/-1.1 |

### TABLE VII

| Total Capacity | | 133,000 ng/ml glass |
|---|---|---|
| Effective Capacity | | |
| Maximum Endotoxin Concentration Allowed In Column Effluent | | Effective Capacity |
| 0.1 ng/ml 1.0 ng/ml | | 49,000 ng/g glass 56,000 ng/g glass |

## TABLE VIII

### Adsorption of Protein on Bare and Coated
### Glass Fiber Materials

| Sample | Protein | Initial Conc. ($\mu$g/ml) | Final Conc. ($\mu$g/ml) | Amount Adsorbed ($\mu$g/g fiber) | Fiber Wt. (mg) |
|---|---|---|---|---|---|
| Bare Glass | Insulin | 6.3 | 6.4 | NDA | 200 |
| Bare Glass | BSA | 11.2 | 12.7 | NDA | 200 |
| Bare Glass | BSA* | 1250 | 1034 | 1080 | 4000 |
| Agarose #1 | BSA** | 2000 | 1795 | 410 | 5000 |
| Agarose #1 | BSA** | 1000 | 959 | 82 | 5000 |
| Agarose #1 | BSA | 11.2 | 10.7 | NDA | 200 |
| Agarose #1 | Insulin | .13 | 14.5 | NDA | 200 |
| Agarose #1 | Insulin | 102 | 103 | NDA | 200 |
| Agarose #3 | Insulin | 13 | 12.3 | NDA | 200 |
| Agarose #3 | Insulin | 102 | 106 | NDA | 200 |
| Agarose #2 | Insulin | 97 | 93.5 | NDA | 100 |
| Agarose #2 | Insulin | 69 | 67 | NDA | 100 |

All samples incubated with 5 ml protein solution except *
where 20 ml was used and ** where 10 ml was used.
NDA - No Detectable Adsorption

### Claims

1. A glass fiber having on its surface a coating comprising a porous hydrophilic matrix derivatized to bind a suitable ligand or biological material.

2. A glass fiber according to claim 1, wherein the porous hydrophilic matrix is selected from agarose, polyacrylamides, dextran and hydroxyalkyl methacrylates.

3. A column packing or chromatographic medium useful for bioseparations comprising a plurality of glass fibers according to claim 1 or claim 2.

4. A biological material composite comprising a ligand or biological material immobilized on a glass fiber according to claim 1 or claim 2.

5. A glass fiber having on its surface a coating comprising a porous hydrophilic matrix comprised of agarose derivatized to bind a suitable ligand or biological material.

6. A glass fiber according to claim 5, wherein the agarose matrix is present in an amount of at least 1% by weight.

7. A glass fiber according to claim 5 or claim 6, wherein the agarose matrix has been applied to the glass fiber surface in the form of a hot melt.

8. A glass fiber according to any one of claims 5 to 7, wherein the agarose matrix has been applied to the glass fiber surface using a coating off-line process.

9. A glass fiber according to any one of claims 5 to 8, wherein the agarose matrix has been activated with cyanogen bromide or sulfonyl chloride.

10. A column packing or chromatographic medium useful for removing toxins in a bioseparation process comprising a plurality of glass fibers according to any one of claims 5 to 9.

11. A biological material composite comprising a ligand or biological material immobilized on a glass fiber according to any one of claims 5 to 9.

12. A composite according to claim 4 or claim 11, wherein the ligand or biological material is selected from animal cells, antibodies, antibiotics, antigens, carbohydrates, bacteria, coenzymes, enzymes, fungi, microbial cells, plant cells, tissue cultures and yeast.

13. A composite according to claim 12, wherein the ligand or biological material comprises an antibiotic.

14. A composite according to claim 13, wherein the antibiotic comprises polymyxin B.

FIG. 1

FIG. 2

EP 0 328 256 A1

# BREAKTHROUGH PROFILE
## ENDOTOXIN ADSORPTION

COLUMN PARAMETERS

FEED CONCENTATION 1700 ng/ml
FLOW RATE 0.5 ml/min
BED VOLUME 25 ml
PACKING WEIGHT 6g GLASS

FIG.3

EP 0 328 256 A1

# BREAKTHROUGH PROFILE
## ENDOTOXIN ADSORPTION

-COLUMN PARAMETERS
-FEED CONCENTRATION 1700ng /ml
-FLOW RATE 0.5 ml /min
-BED VOLUME 25 ml
-PACKING WEIGHT 6g GLASS

FIG. 4

EP 0 328 256 A1

BREAKTHROUGH PROFILE FOR ENDOXIN ADSORPTION
BY
AGAROSE COATED POLYMYXIN ACTIVATED GLASS FIBERS

COLUMN EFFLUENT ENDOTOXIN CONCENTRATION (ng/ml)

COLUMN PARAMETERS
FEED CONCENTRATION 1700 ng/ml
–FLOW RATE 0.5 ml/min
–BED VOLUME 25 ml
–PACKING WEIGHT 6 g GLASS

EXTRAPOLATED BEYOND 450 ml

VOLUME FED TO COLUMN (ml)

FIG.5

EP 0 328 256 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 197 784 (GENET CORP.) * Page 5, line 25 - page 6, line 30; page 7, lines 26-32 * | 1-7,11-13 | B 01 J 20/32 C 12 N 11/00 |
| P,Y | EP-A-0 266 580 (EXCORIM KB) * Page 2, lines 24-33; page 3, line 14; page 3, lines 24-28; example 3, page 4 * | 1-7,11-13 | |
| X | FR-A-2 083 067 (MONSANTO CO.) * Page 2, lines 27-38; page 5, lines 16-28; page 11, line 34 - page 12, line 2; page 18, lines 10-14 * | 1,2,4,12 | |
| A | US-A-4 111 838 (J.R. SCHAEFFER) * Column 2, line 8 - column 3, line 30; column 3, line 59 - column 4, line 2 * | 1-6,9,11,12 | |
| A | EP-A-0 203 049 (LKB-PRODUKTER AB) * Page 1, lines 6-9; page 3, lines 11-17; page 4, line 29 - page 5, line 25 * | 1-3 | TECHNICAL FIELDS SEARCHED (Int. Cl.4) |
| A | WO-A-8 707 531 (ROYAL POSTGRADUATE MEDICAL SCHOOL) * Page 2, line 34 - page 3, line 35; page 5, line 31 - page 6, line 4 * | 5,9-14 | B 01 J C 12 N |

The present search report has been drawn up for all claims

| Place of search | Date of completion of the search | Examiner |
|---|---|---|
| THE HAGUE | 24-05-1989 | HILGENGA K.J. |